# EUROPÄISCHE PATENTANMELDUNG

(11) **EP 3 705 563 A1**
(43) Veröffentlichungstag der Anmeldung: **09.09.2020**
(21) Anmeldenummer: 20154242.0
(22) Anmeldetag: 29.01.2020
(51) Int. Cl.: C12M 1/107, C12M 1/06, C12M 1/00

(54) **VERFAHREN ZUM BEHANDELN VON RESTEN AUS DER VERGÄRUNG SOWIE VORRICHTUNG ZUM VERGÄREN BIOGENER ABFÄLLE**

(30) Priorität: 06.03.2019 DE 102019001500
(71) Anmelder: Martin GmbH für Umwelt- und Energietechnik, 80807 München (DE)
(72) Erfinder: Martin, Ulrich, 81247 München (DE); Ford, Simon, 81539 München (DE); Rupp, Marius, 85540 Haar (DE)
(74) Vertreter: Castell, Klaus

(57) **Zusammenfassung**

In einem Fermenter werden biogene Abfälle anaerob vergoren, um Biogas und einen Gärrest zu erzeugen. Der Gärrest wird mit Wärme getrocknet, die bei der Verbrennung von Biomasse entsteht.

## Beschreibung

Die Erfindung betrifft ein Verfahren, bei dem biogene Abfälle in einem Fermenter anaerob vergoren werden, wobei Biogas und Gärrest erzeugt werden und der Gärrest getrocknet wird. Außerdem betrifft die Erfindung eine Vorrichtung mit einem Fermenter zum Vergären biogener Abfälle und einer Trocknungseinrichtung zum Trockenen des Gärrestes aus dem Fermenter. Insbesondere betrifft die Erfindung die Behandlung biogener Abfälle in einem Fermenter, der als waagerecht aufgestellter Hohlzylinder eine Welle mit Paddeln aufweist, um die biogenen Abfälle im Fermenter zu bewegen.

Biogene Abfälle können beispielsweise aus dem Inhalt der braunen Tonne stammen, in der Bioabfälle aus Haushalten gesammelt werden. Dies sind nasse, organische Abfälle von Lebensmitteln. Biogene Abfälle sind aber auch eher trockene, organische Abfälle beispielsweise aus Gärten und verholzte Rückstände, die als grüne Abfälle bezeichnet werden.

Bei der Vergärung der biogenen Abfälle entstehen prozessbedingt innerhalb von mehreren Wochen Biogas sowie ein fester und ein flüssiger Gärrest. Dabei bildet das Biogas den wertvollsten Bestandteil, da es in der Regel etwa 50 % Biomethan enthält.

Am Fermenterausgang werden feste und flüssige Gärreste entnommen, die in der Regel durch aufwendige Aggregate getrennt werden, sodass ein möglichst trockener, fester Gärrest und ein möglichst feststofffreier, flüssiger Gärrest entstehen. Der feste Gärrest kann unter unterschiedlichen aeroben oder anaeroben Bedingungen kompostiert werden, um anschließend beispielsweise als Blumenerde vermarktet zu werden. Als fester Gärrest entsteht auch das sogenannte Überkorn. Dies sind Holz oder holzartige, grobkörnige Partikel mit einer Partikelgröße von über 40 mm bis 80 mm. Aufgrund seiner holzartigen, organischen Struktur kann das Überkorn nur in geringem Maße vergoren werden und baut sich daher im Fermenter nicht ab. Es wird daher vom Gärrest abgetrennt und separat entsorgt.

Der flüssige Gärrest wird, soweit dies noch möglich ist, in der Landwirtschaft auf Felder ausgebracht.

Das erfindungsgemäße Verfahren dient insbesondere der Trockenvergärung. Dabei wird mit einem deutlich höheren Trockensubstanzgehalt als bei der Nassvergärung gearbeitet. Der Trockensubstanzgehalt kann dabei bei bis zu 28 % GG Trockensubstanz liegen.

Der Erfindung liegt die Aufgabe zugrunde, ein gattungsgemäßes Verfahren in Hinblick auf die energetische Situation und die Art der Stoffströme weiterzuentwickeln und eine Vorrichtung vorzuschlagen, die sich insbesondere auch für das erfindungsgemäße Verfahren eignet.

Diese Aufgabe wird mit einem Verfahren nach Anspruch 1 und einer Vorrichtung nach Anspruch 10 gelöst. Vorteilhafte Ausgestaltungen von Verfahren und Vorrichtungen sind Gegenstand der Unteransprüche.

Der Erfindung liegt die Erkenntnis zugrunde, dass es sinnvoll ist, zumindest einen Teil des Gärrestes zu trocknen, wenn hierfür Wärme aus der Verbrennung von Biomasse verwendet wird. Dies ermöglicht es, grüne Abfälle oder Lebensmittelabfälle oder grüne Abfälle und Lebensmittelabfälle zusammen zu vergären. Der Gärrest wird dann als trockenes, stabiles, lagerfähiges Produkt als Endprodukt aus den Verfahren produziert. Vorzugsweise entsteht bei den erfindungsgemäßen Verfahren kein flüssiger Gärrest.

Besonders vorteilhaft ist es, wenn während des Vergärens der biogenen Abfälle der Trockensubstanzgehalt innerhalb des Fermenters bei 15 % bis 30 % GG liegt. Dies führt zu einer nicht zu nassen Vergärung, während die Feuchtigkeit jedoch so hoch eingestellt bleibt, dass eine anaerobe Vergärung nicht behindert wird.

Das produzierte Biogas kann in einem Blockheizkraftwerk verbrannt werden, wobei Strom und Wärme erzeugt wird. Die dabei produzierte Wärme kann einen Teil der Wärmeversorgung für die Gärresttrocknung liefern. Alternativ kann das produzierte Biogas auch zu Biomethan aufbereitet werden.

Besonders vorteilhaft ist es weiterhin, wenn die Biomasse, die verbrannt wird, um die Wärme für die Trocknung des Gärrestes bereitzustellen, Überkorn aufweist. Dies sind holzartige, grobkörnige Partikel.

Dieses Überkorn kann aus einer beliebigen Anlage zur Aufbereitung biogener Abfälle stammen, insbesondere aus Kompostieranlagen zur Erzeugung von Erde, da dort gröbere Partikel mit Größen oberhalb 40 mm bis 80 mm aussortiert werden müssen.

Dieses Überkorn kann jedoch auch durch Aussieben des im Fermenter erzeugten Gärrestes gewonnen werden. Ein derartiges Überkorn stellt eine günstige Art der Biomasse zur Verfügung, die zur Erzeugung der für die Trocknung des Gärrestes benötigten Wärme verwendet werden kann.

Die Biomasse kann jedoch auch Abfallholz oder getrockneten Gärrest aufweisen. Je nach Verwertbarkeit der einzelnen Stoffströme ermöglicht das erfindungsgemäße Verfahren entweder Stoffe aus dem Verfahren auszuscheiden und zu verwerten oder im Verfahren weiter zu verwerten.

Versuche haben gezeigt, dass es besonders vorteilhaft ist, wenn der Gärrest auf einem Bandtrockner, mit einem Scheibentrockner oder einem mehrstufigen Verdampfer getrocknet wird.

Eine Weiterbildung des Verfahrens sieht vor, dass bei der Verbrennung der Biomasse aus der produzierten Wärme Strom erzeugt wird. Die Wärme kann somit entweder zu einem Teil für die Trocknung und zu einem anderen Teil zur Stromerzeugung verwendet werden oder sie kann auf einem höheren Temperaturniveau zur Stromerzeugung und auf einem niedrigeren Temperaturniveau zur Trocknung verwendet werden.

Weiterhin kann es je nach Verfahrensführung vorteilhaft sein, wenn das Vergären der biogenen Abfälle durch bei der Verbrennung von Biogas oder Biomasse entstandene Wärme unterstützt wird.

Vorrichtungsmäßig wird ein Fermenter zum Vergären biogener Abfälle mit einer Trocknungseinrichtung zum Trocknen des Gärrestes kombiniert und diese Kombination wird mit einer Verbrennungseinrichtung für die Verbrennung von Biomasse kombiniert, um Wärme der Verbrennungseinrichtung für die Trocknungseinrichtung zu nutzen.

Dabei kann als Fermenter ein Hohlzylinder mit mehreren Metern Durchmesser verwendet werden, der eine zentrale, mittlere Welle aufweist, von der radial Paddel abstehen, um in dem nur teilweise mit biogenen Abfällen gefüllten Fermenter die Abfälle zu bewegen, um eine kontinuierliche Durchmischung zu erreichen.

Zwei vorteilhafte Verfahrensvarianten sind in der Zeichnung dargestellt und werden im Folgenden näher beschrieben. Es zeigt
- Figur 1: schematisch als Blockdiagramm die Behandlung biogener Abfälle mit Verbrennung des Überkorns zur Generierung von Wärme für die Trocknung und
- Figur 2: ein Verfahren gemäß Figur 1 mit zusätzlichen Einrichtungen.

Bei den in den Figuren gezeigten Verfahren werden biogene Abfälle 1 in einem Fermenter 2 anaerob vergoren. Dabei entsteht ein Gärrest 3 und ein Biogas 4. Der Gärrest 3 wird anschließend einer Trocknung 5 in einer nicht gezeigten Trocknungseinrichtung wie beispielsweise einem Bandtrockner, einem Scheibentrockner oder einem mehrstufigen Verdampfer zugeführt.

Bei der Trocknung 5 entstehen Brüden 6 (verdampftes Wasser) und ein getrockneter Gärrest 7.

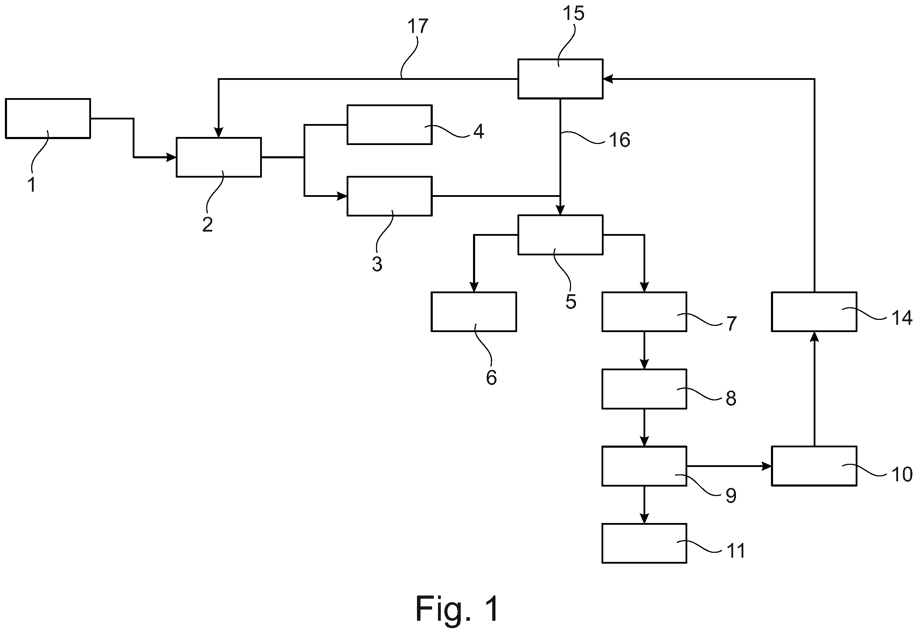

Der getrocknete Gärrest 7 kann wie in Figur 1 gezeigt zunächst einer Kompostierung 8 zugeführt werden. Danach kann bei einer Siebung 9 Überkorn 10 abgetrennt werden. Der feinere Rest der Siebung 9 ist dann Kompost 11, der beispielsweise zu Erde weiterverarbeitet werden kann.

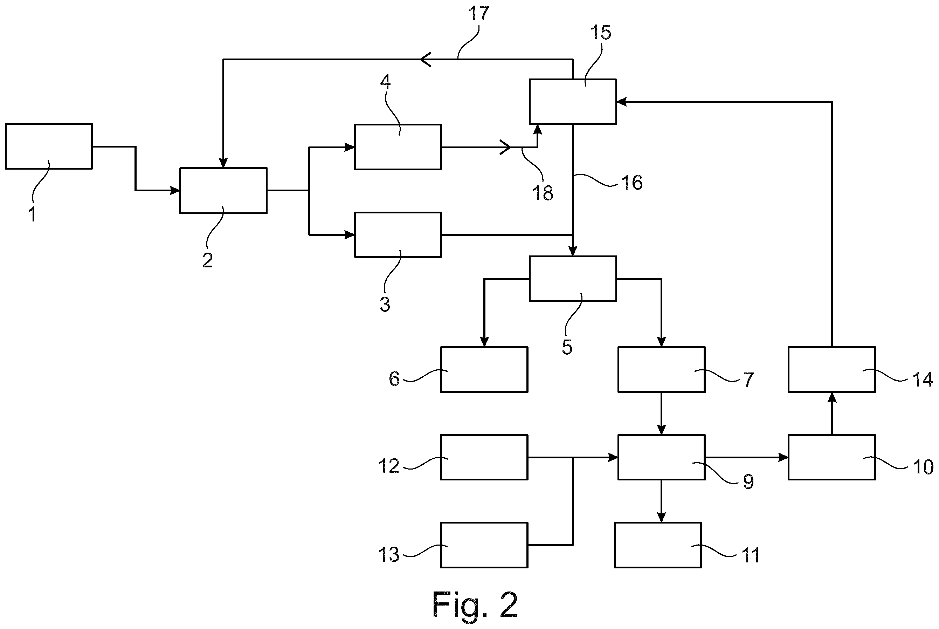

Der getrocknete Gärrest 7 kann jedoch auch wie in Figur 2 gezeigt ohne vorrangehende Kompostierung gesiebt werden, um Überkorn 10 abzutrennen. Dann bildet der getrocknete Gärrest nach der Siebung den Kompost 11.

Die Masse des Überkorns kann wie in Figur 2 gezeigt, erhöht werden, wenn neben dem getrockneten Gärrest 7 auch Biomasse 12 von Kompostieranlagen am gleichen Standort oder Biomasse 13 von Kompostieranlagen an fremden Standorten, vorzugsweise über die Siebung 9 zusammen mit dem Überkorn 10 als gesamte Biomasse 14 zur Verbrennung einem Blockheizkraftwerk 15 zugeführt wird.

Im Blockheizkraftwerk 15 entsteht Wärme 16, mit der die Trocknung 5 des Gärrestes 3 unterstützt wird. Außerdem erzeugt das Blockheizkraftwerk 15 Strom 17, mit dem beispielsweise der Fermenter 2 angetrieben werden kann. Die Verbrennung im Blockheizkraftwerk 15 kann auch durch Biogas 4 aus dem Fermenter 2 entsprechend dem Gasstrom 18 unterstützt werden.

Der bei der Trocknung 5 entstehende Brüden 6 kann stark geruchsbelastet sein. Daher ist es vorteilhaft, wenn dieser Brüden 6 vor dem Entweichen in die Umgebung gereinigt wird. Eine sinnvolle Verwendung des Brüdens 6, die auch die Geruchbelastung reduzieren kann, ist das Einleiten in eine beliebige Kompostieranlage oder sogar in die Kompostieranlage 12. Kumulativ oder alternativ kann der Brüden 6 auch dem Fermenter 2 zugeführt werden. Er kann somit für eine direkte oder eine indirekte Erwärmung des Fermenters und/oder zur Befeuchtung im Fermenter dienen.

## Patentansprüche

1. Verfahren, bei dem biogene Abfälle (1) in einem Fermenter (2) anaerob vergoren werden, wobei Biogas (4) und Gärrest (3) erzeugt werden, und der Gärrest (3) getrocknet wird, ***dadurch gekennzeichnet, dass*** zumindest ein Teil der für die Trocknung (5) des Gärrestes (3) benötigten Wärme (16) durch eine Verbrennung von Biomasse (14) bereitgestellt wird.

2. Verfahren nach Anspruch 1, ***dadurch gekennzeichnet, dass*** während des Vergärens der biogenen Abfälle (1) der Trockensubstanzgehalt innerhalb des Fermenters bei 15 bis 30 % GG liegt.

3. Verfahren nach einem der vorhergehenden Ansprüche, ***dadurch gekennzeichnet, dass*** das erzeugte Biogas (4) in einem Blockheizkraftwerk (15) verwertet wird und Wärme (16) des Blockheizkraftwerkes (15) für die Trocknung (5) des Gärrestes (3) verwendet wird.

4. Verfahren nach einem der vorhergehenden Ansprüche, ***dadurch gekennzeichnet, dass*** die Biomasse (14), die verbrannt wird, um die Wärme für die Trocknung (5) des Gärrestes (3) bereit zu stellen, Überkorn (10) aufweist.

5. Verfahren nach Anspruch 4, ***dadurch gekennzeichnet, dass*** das Überkorn (10) durch Absieben (9) des im Fermenter erzeugten Gärrestes (3) gewonnen wird.

6. Verfahren nach einem der vorhergehenden Ansprüche, ***dadurch gekennzeichnet, dass*** die Biomasse (14) Abfallholz oder getrockneten Gärrest (3) aufweist.

7. Verfahren nach einem der vorhergehenden Ansprüche, ***dadurch gekennzeichnet, dass*** der Gärrest (3) auf einem Bandtrockner, mit einem Scheibentrockner oder einem mehrstufigen Verdampfer getrocknet wird.

8. Verfahren nach einem der vorhergehenden Ansprüche, ***dadurch gekennzeichnet, dass*** bei der Verbrennung der Biomasse (14) aus der produzierten Wärme Strom (17) erzeugt wird.

9. Verfahren nach einem der vorhergehenden Ansprüche, ***dadurch gekennzeichnet, dass*** das Vergären der biogenen Abfälle (1) durch bei der Verbrennung von Biogas (3) oder Biomasse (14) entstandene Wärme (16) unterstützt wird.

10. Vorrichtung mit einem Fermenter zum Vergären biogener Abfälle (1) und einer Trocknungseinrichtung zum Trocknen (5) des Gärrestes (3) aus dem Fermenter, ***dadurch gekennzeichnet, dass*** sie eine Verbrennungseinrichtung für die Verbrennung (15) von Biomasse (14) aufweist, die derart mit der Trocknungseinrichtung in Verbindung steht, dass Wärme (16) der Verbrennungseinrichtung für die Trocknungseinrichtung genutzt werden kann.

11. Vorrichtung nach Anspruch 10, ***dadurch gekennzeichnet, dass*** der Fermenter ein waagerecht aufgestellter Hohlzylinder ist, der eine Welle mit Paddeln aufweist, um biogene Abfälle (1) im Fermenter zu bewegen.
